# EUROPEAN PATENT APPLICATION

(11) **EP 4 324 437 A1**
(43) Date of publication of application: **21.02.2024**
(21) Application number: 23382148.7
(22) Date of filing: 17.02.2023
(51) Int. Cl.: A61F 5/56

(54) **MANDIBULAR ADVANCEMENT DEVICE FOR SLEEP APNOEA AND HYPOPNOEA**

(30) Priority: 17.08.2022 ES 202231368 U
(71) Applicant: Giromed Institute, S.L.P., 17003 Girona (ES)
(72) Inventor: ROLDAN SÁNCHEZ, Juan, Girona (ES)
(74) Representative: Pons

(57) **Abstract**

The present invention relates to a mandibular advancement device (1) for sleep apnoea and hypopnoea, comprising an upper splint (2), a lower splint (3), an adjustment mechanism for adjusting the position of the lower splint (3) relative to the upper splint (2), comprising two stops (5), arranged on the outer side faces of the upper splint (2), and two wings (4), arranged on the outer posterolateral regions of the lower splint (3), so that when the user closes their mouth, the wings (4) are in front of the stops (5), closer to the front area of the user's mouth, the user's lower arch being forward of the upper arch. The device (1) may further comprise a sensor module (14) for monitoring the user.

## Description

### OBJECT OF THE INVENTION

The object of the present invention is a customised smart mandibular advancement device (smart MAD) for the treatment of sleep apnoea or hypopnoea syndrome (OSAS). The device is intended to achieve a clinical improvement in symptoms that will have a direct impact on the user's quality of life. The device is associated with a charging and data unit intended to record certain biometric parameters of interest to the user and healthcare professionals that will be measured when the mandibular advancement device is used.

In this sense, the use of personalised medicine makes it possible to properly manage the treatment, adherence, compliance and follow-up of patients affected by OSAS in order to reduce the morbidity and mortality associated with the disease. This will have a direct impact on reducing excessive healthcare expenditure generated by patients with OSAS who are not treated on time or have low treatment compliance.

### BACKGROUND OF THE INVENTION

OSAS (Obstructive Sleep Apnoea Syndrome) is a chronic disease characterised by repetitive obstruction of the upper airways at the pharyngeal level during sleep, with complete (apnoea) or partial (hypopnoea) cessation of airflow. This is caused by an anatomical and functional alteration of the upper airways that makes said airways more collapsible than in healthy subjects.

Recurrent apnoea and hypopnoea lead to repeated oxyhaemoglobin desaturations, causing intermittent hypoxia, microarousals with sleep fragmentation, and significant intrathoracic pressure oscillations due to increased inspiratory effort in each apnoeic event. This leads to sudden increases in sympathetic tone, heart rate and blood pressure, responsible for sympathetic activation and endothelial dysfunction.

OSAS is a highly prevalent disease. Different epidemiological studies show a prevalence of the disease of 8% in the general population over 40 years of age. This prevalence clearly increases with age, reaching up to 26% in men and 21% in women in the population over 65 years of age. In Spain, it is estimated that between 5 and 7 million people suffer from OSAS.

Many studies have established OSAS as an added risk factor for cardiovascular disease and an increase in the incidence of traffic accidents. Furthermore, it is associated with cognitive disorders and impaired quality of life, and in children it affects learning and behaviour. For all these reasons, OSAS is considered a major public health problem. It represents the third most frequent respiratory disease after bronchial asthma and chronic obstructive pulmonary disease, and it is the most frequent alteration that occurs during sleep, as well as the main cause of chronic hypoxia in humans, due to intermittent hypoxemia.

For this reason, proper management in the treatment and follow-up of patients affected by OSAS is very important in order to reduce morbidity and mortality. Furthermore, it is necessary to take into account the excessive health expenditure generated by patients with OSAS who are not treated in a timely and adequate manner.

Currently, there is a range of therapies used for the treatment of sleep apnoea and hypopnoea:
- Continuous positive airway pressure (CPAP). It consists of a non-invasive mechanical ventilation device that applies positive pressure through a nasal mask, which prevents the upper airways from collapsing. For some patients, the improvement in quality of sleep and quality of life are immediate and in the long term it reduces mortality associated with OSAS. However, the biggest problem with CPAP is adherence to treatment, since only 60% of patients use it after one year of follow-up, adherence being better in those who are older, with more severe problems, drowsier, with high blood pressure and it being poorly tolerated in asymptomatic snorers.
- Surgical treatment. It has not been shown to be effective, due to adverse effects and the low success rate, less than 50%, in reducing the apnoea index.
- Mandibular repositioning. The aim of intraoral appliances is defined as the modification of the anatomical position of orofacial structures to eliminate obstruction of the airways, which makes it possible to improve the symptoms of snoring and apnoea. The indication for these devices is the treatment of snoring and mild or moderate OSAS in individuals with severe symptoms who do not tolerate CPAP treatment. The aim is to reduce episodes of sleep apnoea, increase the quality of oxygen in the blood and reduce the risk of cardiovascular disease.

### DESCRIPTION OF THE INVENTION

The mandibular advancement device object of the present invention achieves an improvement in the quality of life of users who suffer from sleep apnoea or hypopnoea. The device makes it possible to adapt the mechanical and kinetic behaviour of a user's jaws while, in one embodiment of the invention, it allows biometric parameters such as heart rate (HR), oxygen saturation (SaO2), and the position in which the user sleeps to be recorded. All these data are of interest in relation to the user's status.

The device may further comprise a recording and charging unit, which records and stores the obtained parameters of interest, and these parameters can be sent to a specialist for analysis.

Specifically, the mandibular advancement device comprises two independent splints, an upper splint for the upper arch and a lower splint for the lower arch of a user. Both splints are coupled by an advancement adjustment mechanism located on the back, made of a highly resistant biocompatible material that supports a high degree of bending, load and resistance to abrasion. The mechanism arranged between both splints allows for more optimal mandibular advancement through the use of an adjustment mechanism, which can be adjusted to the millimetre.

The mandibular advancement adjustment mechanism is positioned on the outer sides of each of the splints. Specifically, there are stops located on the outer side faces of the upper splint, between the first and second molars. These stops can be coupled to and uncoupled from the upper splint.

With respect to the lower splint, there are wings are arranged on the outer posterolateral regions of the same, between the first and second molars. When the user closes their mouth, the wings are in front of the stops, i.e., closer to the front area of the mouth. The stops prevent the wings from advancing towards the back of the mouth, so that the user's lower jaw is forward of the upper jaw. Thus, the adjustment mechanism allows for optimal articulation and positioning between both splints.

Furthermore, since the stops are independent of the upper splint, they can be easily removed and replaced, so that their size is customised based on the desired position of the patient's jaws, adapting it to each one and modifying it depending on the stage of treatment.

In this way, when a user puts on the mandibular advancement device, the lower jaw is more forward of the upper jaw, adopting a more physiological position that prevents episodes of sleep apnoea or hypopnoea.

Therefore, the adjustment mechanism allows this position to be adjusted depending on the phase of the treatment that the user is in. Furthermore, since the adjustment mechanism can make adjustments to the millimetre, the ideal position for each user will be defined by the sleep specialist to correct apnoea.

From the defined position, the mandibular advancement device will not allow the lower jaw to retract. However, the mandibular advancement device will allow movements of laterality (lateral displacement of one jaw relative to the other) and opening to be carried out, since the adjustment mechanism can be articulated relative to the splints due to the design of the wings, which achieves greater user comfort. This is a great advantage in terms of the user's tolerance to the device, as well as the follow-up of the treatment, avoiding its abandonment.

Moreover, and in order to monitor a series of parameters of interest with a view to carrying out the treatment and follow-up of the user, the mandibular advancement device may further comprise a sensor module, preferably embedded in one side of the lower splint. A first element of this module is a controller, intended to manage said sensor module. Furthermore, it comprises a rechargeable power module.

On the other hand, in the sensor module there is an identification sensor, which is a sensor intended to know when the user is using the mandibular advancement device and it is preferably an RFID radio frequency identification sensor.

Furthermore, the sensor module may comprise a blood oxygen saturation sensor or pulse oximeter and a heart rate sensor. Specifically, in one embodiment, both sensors are optical sensors, being able to extract the heart rate signal and blood oxygen saturation. These sensors are based on already existing technology found in hospital instrumentation, which measures blood flow to the ends of the fingers or even to the ear.

Finally, the sensor module may further comprise a position sensor, preferably an accelerometer, which makes it possible to know if the user is sleeping face up, face down, on the right side or left side while using the device. All the described sensors are connected to the controller and powered by the power module.

All the information collected in the sensor module is integrated into a mathematical algorithm in the controller and helps to know the user's health status in real time, which allows the specialist to make decisions regarding the most appropriate treatment.

Furthermore, also connected to the controller and powered by the power module, the sensor module comprises a communications module, intended to send the data collected in the sensor module to an external system or other device.

Specifically, the communications module can send the collected data directly to an external system, such as a computer or mobile phone, or upload it directly to the cloud, so that the specialist can analyse it and determine if the user's treatment is correct or if some readjustment should be made to the mandibular advancement device.

Alternatively, the mandibular advancement device may comprise a data recording and charging unit. In one embodiment, the communications module sends the collected data to the data recording unit, which stores it and sends it to an external system, such as a computer, mobile phone, tablet or the cloud, for analysis by the specialist.

The data recording unit may comprise an alert generation module, capable of alerting the status of the mandibular advancement device, either to the user himself or to the specialist through the data sent.

Furthermore, the data recording unit can wirelessly recharge the power module, so that when the user puts the device on at night before sleeping, it is fully charged.

In summary, the mandibular advancement device, object of the present invention, takes into account the mechanical and kinetic behaviour of users' jaws through a comprehensive analysis of the jaw, and it has the following advantages:
- it establishes an effective alternative for the treatment of OSAS (Obstructive Sleep Apnoea Syndrome),
- it allows the efficacy and safety of the mandibular advancement device to be evaluated through the analysis of data from its sensors, said data which is sent to the specialist either directly or alternatively through the data recording and charging unit,
- it improves the user's quality of life and the level of satisfaction through close and customised follow-up,
- it improves the treatment of the user, since a more constant follow-up of their evolution can be carried out, and not every six or twelve months as is currently done,
- it reduces displacement and waiting times in hospital queues, since the user does not have to visit the specialist a number of times, and
- it improves the patient's ability to self-manage the disease, since they will have detailed information about their data from anywhere.

### DESCRIPTION OF THE DRAWINGS

As a complement to the description provided and for the purpose of helping to make the features of the invention more readily understandable, in accordance with a practical preferred exemplary embodiment thereof, said description is accompanied by a set of drawings which, by way of illustration and not limitation, represent the following:
Figure 1.- Shows a general view of the mandibular advancement device.
Figure 2.- Shows a view of the upper splint.
Figure 3.- Shows a view of the lower splint.
Figure 4.- Shows a view of the adjustment mechanism.
Figure 5.- Shows a general diagram of the mandibular advancement device in communication with the data recording unit and data delivery unit to the specialist.
Figure 6.- Shows a schematic representation of the sensor module.

### PREFERRED EMBODIMENT OF THE INVENTION

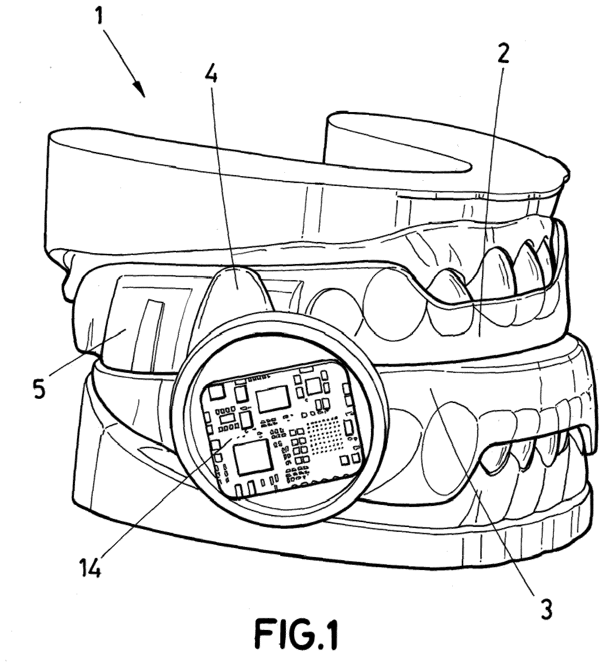

A preferred embodiment of the mandibular advancement device (1), object of the present invention, is described below with the aid of figures 1 to 6.

The mandibular advancement device (1) for sleep apnoea and hypopnoea, shown in figure 1, allows the lower jaw to be repositioned relative to the upper jaw of a user, moving it forward, as well as collecting a series of physiological data of interest from said user.

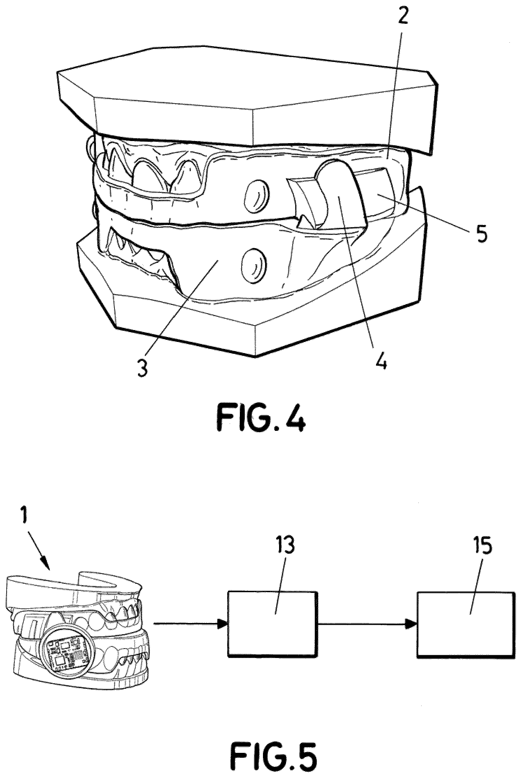

Furthermore, the mandibular advancement device (1) comprises a recording and charging unit (13) so that the obtained data of interest is recorded, and this data can be sent to an external system (15), such as the cloud, a computer or a mobile phone, so that a specialist can proceed to analyse it, as shown in figure 5.

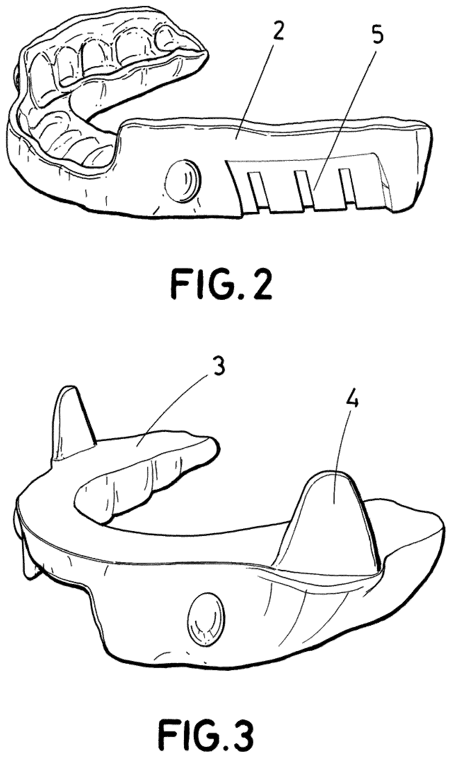

Specifically, the mandibular advancement device (1) comprises two independent splints (2, 3), an upper splint (2) for the upper arch, which is shown in figure 2, and a lower splint (3) for the lower arch of the user, which is shown in figure 3. Both splints (2, 3) are preferably made of a highly resistant biocompatible material that supports a high degree of bending, load and resistance to abrasion.

Furthermore, as shown in detail in figure 4, the device (1) comprises an adjustment mechanism, which allows the position of the lower splint (3) to be adjusted relative to the upper splint (2) so that the user's lower jaw is forward of their upper jaw.

Both the splints (2, 3) and the adjustment mechanism are made of the highest quality biocompatible and hypoallergenic acrylic material. This material provides great comfort and permeability to the user.

In particular, the mandibular advancement adjustment mechanism is positioned on the outer sides of each of the splints (2, 3). Specifically, there are stops (5) located on the outer side faces of the upper splint (2), between the first and second molars, as shown in detail in figure 2, said stops protruding laterally from said upper splint (2). These stops (5) can be coupled to and uncoupled from the upper splint (2). For this, the upper splint (2) comprises on an outer face, in the area where the stops (5) are arranged, first guides which can be coupled to second guides arranged on the inner face of the stops (5).

With respect to the lower splint (3), there are wings (4) are arranged on the outer posterolateral regions of the same, between the first and second molars, as shown in figure 3. When the user closes their mouth, the wings (4) are in front of the stops (5), i.e., closer to the front area of the mouth. The stops (5) prevent the wings (4) from advancing towards the back of the mouth, so that the user's lower jaw is forward of the upper jaw. Thus, the adjustment mechanism allows for optimal articulation and positioning between both splints (2, 3), as shown in figure 4.

Furthermore, since the stops (5) are independent of the upper splint, they can be easily removed and replaced, so that their size is customised based on the desired position of the patient's jaws, adapting it to each one and modifying it depending on the stage of treatment.

In this way, when a user puts on the mandibular advancement device (1), the lower jaw is more forward of the upper jaw, adopting a more physiological position that prevents episodes of sleep apnoea or hypopnoea.

From the defined position, the mandibular advancement device (1) will not allow the jaw to retract. However, the device (1) will allow movements of laterality (lateral displacement of one jaw relative to the other) and opening to be carried out. In more detail, it allows carrying out the movements of:
- Anterior/posterior displacement. From the resting position of the jaw, the lower jaw is allowed to move forward. Anterior movement must not be allowed. This movement is the one that will be adjusted depending on the user's physiology.
- Lateral/medial displacement. Taking the contact point of the two lower incisors as the point of reference, movement must be allowed between both jaws on both sides of the point of reference. This movement cannot be adjusted.
- Opening. During the use of the mandibular advancement device (1), the distance between the lower incisors and the upper incisors must be at least 2 to 4 mm. The device (1) must allow a greater distance as long as it does not make it impossible for the user to move the jaw forward. This movement may not be adjusted.

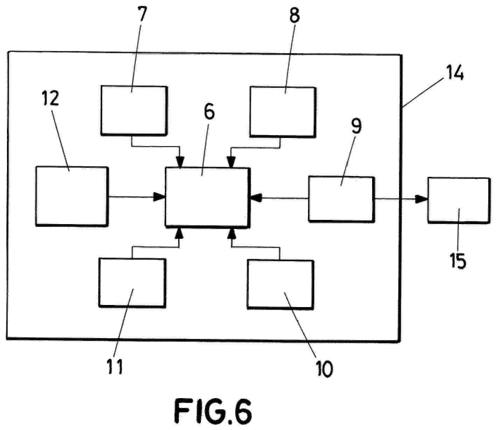

Moreover, and in order to monitor a series of parameters of interest with a view to carrying out the treatment and follow-up of the user, the mandibular advancement device (1) comprises a sensor module (14), which is shown in figure 6. A first element of this module is a controller (6), intended to manage the rest of the elements. Furthermore, it comprises a rechargeable power module (7).

On the other hand, the sensor module (14) comprises an identification sensor (12), which is a sensor intended to know when the user is using the mandibular advancement device (1) and it is preferably an RFID radio frequency identification sensor, which personally identifies the mandibular advancement device (1) and associates it with the user.

Furthermore, the sensor module (14) comprises a blood oxygen saturation sensor (8) or pulse oximeter and a heart rate sensor (10). Specifically, in one embodiment, both sensors are optical sensors intended to capture blood flow in the palate, being able to extract the heart rate signal and blood oxygen saturation.

Finally, the sensor module (14) comprises a position sensor (11), preferably an accelerometer, capable of measuring the position and evaluating the user's activity while sleeping, allowing proper recording of the evolution of the user's disease, obtaining a detail of the impact of apnoea measured continuously. All the sensors described are connected to the controller (6) and powered by the power module (7).

Furthermore, also connected to the controller (6) and powered by the power module (7), the sensor module (14) comprises a communications module (9), intended to send the data collected in the sensor module (14) to an external system (15) or other device, as reflected in figure 6.

Specifically, the communications module (9) can send the collected data directly to an external system (15), such as a computer, or upload it directly to the cloud, so that the specialist can analyse it and determine if the user's treatment is correct or if some readjustment should be made to the mandibular advancement device (1).

All the described elements of the sensor module (14) are placed on a support, such as the one shown in figure 1, which is preferably embedded in the lower splint (3).

Moreover, as indicated, the mandibular advancement device (1) object of the present invention may comprise a data recording and charging unit (13), as indicated in figure 5. The communications module (9) sends the collected data to said data recording and charging unit (13), which stores it and sends it to an external system (15), such as a computer or the cloud, for analysis by the specialist.

Furthermore, the data recording and charging unit (13) wirelessly recharges the power module (7), so that when the user puts the splints (2, 3) on at night before sleeping, it is fully charged.

Specifically, the data recording and charging unit (13) comprises a data sending module based on GSM communication (Global System for Mobile Communications). Furthermore, the data sending module may also use NFC (Near Field Communication), Wi-Fi and Bluetooth communication. In addition, the data recording and charging unit (13) is charged via USB-C.

The data recording and charging unit (13) operates in such a way that, firstly, it checks that the splints (2, 3) are present. If they are, secondly, it checks whether there is data collected in the sensor module (14) to download. If so, said data is sent to an external device (15) or to the cloud.

## Claims

1. A mandibular advancement device (1) for sleep apnoea and hypopnoea, **characterised in that** it comprises:
- an upper splint (2) for the upper arch of a user,
- a lower splint (3) for the lower arch of the user,
- an adjustment mechanism for adjusting the position of the lower splint (3) relative to the upper splint (2), the adjustment mechanism comprising:
- two stops (5), arranged on the outer side faces of the upper splint (2),
- two wings (4), arranged on the outer posterolateral regions of the lower splint (3), so that when the user closes their mouth, the wings (4) are in front of the stops (5), closer to the front area of the user's mouth, the user's lower arch being forward of the upper arch.

2. The device (1) of claim 1, wherein the stops (5) can be decoupled from the upper splint (2).

3. The device (1) of claim 1, further comprising a sensor module (14), comprising:
- a controller (6),
- a power module (7), associated with the controller (6),
- an identification sensor (12), associated with the controller (6), and
- a communications module (9), associated with the controller (6) and capable of sending the data collected in the controller (6) to an external system (17).

4. The device of claim 3, wherein the sensor module (14) further comprises a blood oxygen saturation sensor (8) associated with the controller (6).

5. The device of claim 3, wherein the sensor module (14) further comprises a heart rate sensor (10) associated with the controller (6).

6. The device of claim 3, wherein the sensor module (14) further comprises a position sensor (11), associated with the controller (6).

7. The device of claim 3, wherein the identification sensor (12) of the mandibular advancement device (1) is an RFID radio frequency identification sensor.

8. The device of claims 4 and 5, wherein the blood oxygen saturation sensor (8) and the heart rate sensor (10) of the mandibular advancement device (1) are optical sensors capable of capturing the percentage of blood that carries oxygen.

9. The device of claim 6, wherein the position sensor (11) of the mandibular advancement device (1) is an accelerometer.

10. The device of claim 1, further comprising a data recording and charging unit (13), associated with the power module (7) and with the communications module (9) and capable of storing and transmitting the data collected by the controller (6) to an external system (15), as well as recharging the power module (7) of the mandibular advancement device (1) when not in use.
